# EUROPÄISCHE PATENTSCHRIFT

(11) **EP 1 568 934 B1**
(45) Veröffentlichungstag und Bekanntmachung des Hinweises auf die Patenterteilung: **30.05.2012**
(21) Anmeldenummer: 04004600.5
(22) Anmeldetag: 28.02.2004
(51) Int. Cl.: F21S 8/00, F21V 21/40, F21Y 101/02

(54) **Operationsleuchte**
Surgical light
Lampe chirurgicale

(43) Veröffentlichungstag der Anmeldung: 31.08.2005
(73) Patentinhaber: TRUMPF Medizin Systeme GmbH + Co. KG, 07318 Saalfeld (DE)
(72) Erfinder: Rudolf, Marka, Dr., 81479 München (DE)
(74) Vertreter: Feldmeier, Jürgen

(56) Entgegenhaltungen:
- WO-A-03/019072
- FR-A- 1 311 965
- GB-A- 819 836
- JP-A- 60 119 936
- US-A1- 2003 014 834

## Beschreibung

Die Erfindung betrifft eine Operationsleuchte mit einem Leuchtenkörper zur Aufnahme von Leuchtmitteln.

Eine derartige Operationsleuchte ist beispielsweise durch die DE 198 38 627 A1 bekannt geworden.

Die bekannte Operationsleuchte weist wenigstens eine Strahlungsquelle und wenigstens zwei Reflektoren auf. Zur Bildung eines schattenarmen Beleuchtungsfeldes trifft aus der Operationsleuchte austretende Strahlung mit wenigstens zwei unterschiedlichen Einfallswinkeln auf das Beleuchtungsfeld auf. Dabei sind die Einfallswinkel der Strahlung durch Änderung des Abstandes zwischen wenigstens zwei Reflektoren oder zwischen einem Reflektor und der Strahlungsquelle einstellbar.

Die applikationsbezogene Veränderung der Leuchtfeldform (die auf dem Operationstisch beleuchtete Fläche) ist bei einer Operationsleuchte nach der Lehre des Standes der Technik nicht vorgesehen.

Bestehende Operationsleuchten weisen eine feste Lichtverteilung auf. Operationsleuchten müssen vielfältigen Beleuchtungssituationen gerecht werden. Beispielsweise wird bei großflächigen Wunden zur Vermeidung von Schatten viel Licht vom Rand der Leuchte benötigt, um Hindernisse zu unterleuchten. Im Fall von Wunden mit kleinem Durchmesser und großer Tiefe, wird Licht aus der Mitte der Leuchte erforderlich. Diese Extremsituationen werden mit gängigen Operationsleuchten schlecht abgedeckt, da bekannte Operationsleuchten immer einen Kompromiss in der Lichtverteilung aufweisen.

In der US-Patentanmeldung US 2003/0014834 A1 ist ein Handgriff für eine Operationsleuchte offenbart, in den eine Lichtquelle integriert ist. Die Lichtquelle sendet einen Lichtstrahl aus dem Handgriff, der auf dem Operationsfeld einen Lichtpunkt erzeugt, mit dessen Hilfe Operationsleuchten genauer ausgerichtet werden können.

Heute üblich ist daher der Einsatz von Kopfleuchten oder von in die Wunde eingebrachten Lichtleitfasern. Nachteilig ist aber, dass die Kopfleuchte oder die Lichtfaser den Operateur einschränken (unergonomische Kopfhaltung des Operateurs) bzw. die Lichtleistung durch Verunreinigung und Hygieneprobleme bei direktem Wundkontakt verringern.

Der Anmelder hat sich die Aufgabe gestellt, eine Operationsleuchte zu schaffen, welche eine verbesserte Ausleuchtung tiefer Wunden mit geringem Durchmesser ermöglicht.

Diese Aufgabe wird durch eine Operationsleuchte gemäß Patentanspruch 1 gelöst. Das Grundprinzip besteht darin, eine Lichtquelle im Zentrum der Operationsleuchte getrennt von der eigentlichen Lichtquelle anzusteuern und die Helligkeit im Zentrum der Operationsleuchte zu verändern, d.h. zu erhöhen. Zur technischen Verwirklichung des Grundprinzips kommen im 20 Wesentlichen drei Ausführungsformen in Betracht. Erstens kann die Steuereinrichtung der Operationsleuchte diejenigen Leuchtmittel im Zentrum der Operationsleuchte unabhängig von den anderen ansteuern und die Helligkeit im Zentrum unabhängig vom Rest der Operationsleuchte erhöhen. Zweitens kann eine separate Lichtquelle im Zentrum der Operationsleuchte vorgesehen sein. Drittens kann eine Zusatzlichtquelle im Handgriff, der sich im Zentrum der Operationsleuchte befindet angeordnet werden. Vorteilhafterweise werden als Leuchtmittel LEDs verwendet. Fünf bevorzugte Ausführungsbeispiele der Erfindung sind in der Zeichnung schematisch dargestellt und werden nachfolgend mit Bezug zu den Figuren der Zeichnung näher erläutert. Es zeigt:
- **Fig. 1**: eine Seitenansicht einer Operationsleuchte;
- **Fig. 2**: eine perspektivische Ansicht einer ersten eingeschalteten Operationsleuchte mit erfindungsgemäß vorgesehener Tiefenausleuchtung;
- **Fig. 3**: eine perspektivische Ansicht einer zweiten eingeschalteten Operationsleuchte mit erfindungsgemäß vorgesehener Tiefenausleuchtung;
- **Fig. 4**: eine perspektivische Detailansicht eines Handgriffs der Operationsleuchte nach Fig. 3;
- **Fig. 5**: eine perspektivische Ansicht einer dritten eingeschalteten Operationsleuchte mit erfindungsgemäß vorgesehener Tiefenausleuchtung;
- **Fig. 6**: eine perspektivische Ansicht einer vierten eingeschalteten Operationsleuchte mit erfindungsgemäß vorgesehener Tiefenausleuchtung;
- **Fig. 7**: eine perspektivische Ansicht einer fünften eingeschalteten Operationsleuchte mit erfindungsgemäß vorgesehener Tiefenausleuchtung.

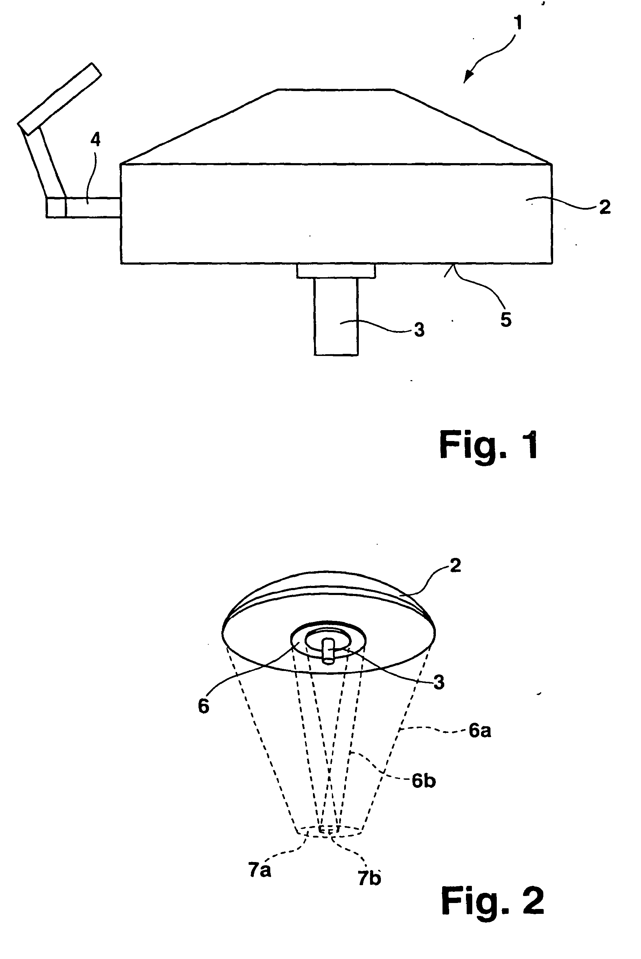

Aus der Seitenansicht einer Operationsleuchte **1** (**Fig. 1**) ist deren prinzipieller an sich bekannter Aufbau ersichtlich. Die Operationsleuchte 1 umfasst einen Leuchtenkörper **2,** welcher in seinem Innenraum in der Fig. 1 nicht sichtbare Leuchtmittel aufweist. Der Leuchtenkörper 2 ist über einen in der Fig. 1 nicht vollständig gezeigten Schwenkarm schwenkbar an einer stationären Halterung an einer Decke oder einer Wand eines Gebäudes oder mobilen Einheit befestigt. Der Schwenkarm ist aus mehreren über Gelenke miteinander verbundenen Elementen aufgebaut. Ein mit der Operationsleuchte 1 fest verbundenes Element **4** des Schwenkarms ist in der Fig. 1 lediglich angedeutet. Daher lässt sich die Operationsleuchte 1 in X-, Y-, Z-Richtung dreidimensional bewegen und schwenken. Ein an dem Leuchtenkörper 2 angebrachter Handgriff **3** ermöglicht die Positionierung der Operationsleuchte an beliebiger Stelle über einem Operationstisch. Der Handgriff 3 ist lösbar an der Unterseite **5** der Operationsleuchte angebracht. Er kann im Zentrum oder außerhalb der Lichtaustrittsfläche positioniert sein.

An der Unterseite 5 tritt Licht aus, um die Operationsstelle auszuleuchten.

**Fig. 2** zeigt, dass eine ringförmige, aus mehreren LEDs bestehende Lichtquelle 6 an der Lichtaustrittsfläche des Leuchtenkörpers 2 rund um den Rand des sterilisierbaren Handgriffes 3 angebracht ist. Durch die zuschaltbare Lichtquelle **6** wird mittels zusätzlicher Lichtstrahlen **6b** ein Lichtfeld **7b** erzeugt, welches ein durch die Lichtstrahlen **6a** erzeugtes Lichtfeld **7a** der Operationsleuchte unterstützt und die Tiefenausleuchtung im Zentrum des Lichtfeldes 7a verbessert. Die Lichtstrahlen der Lichtquelle 6 sind auf den Mittelpunkt des Lichtfeldes 7a fokussiert. Die Intensität der Lichtquelle 6 kann entsprechend geregelt werden, um die Tiefenausleuchtung zu optimieren.

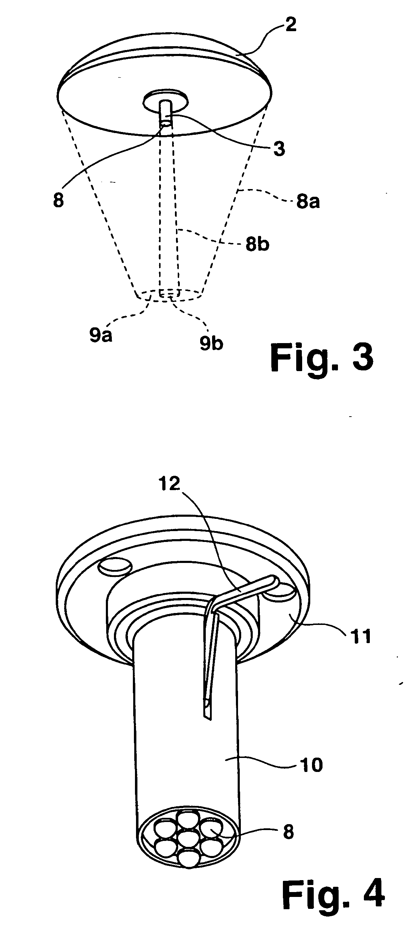

Gemäß einer in **Fig. 3** dargestellten weiteren Ausführungsform sind mehrere zuschaltbare LEDs als zusätzliche Lichtquelle **8** im zentralen sterilisierbaren Handgriff 3 untergebracht. Aus der Lichtquelle 8 treten Lichtstrahlen **8b** aus, so dass im Zentrum eines Lichtfeldes **9a,** welches durch die Lichtstrahlen **8a** erzeugt wird, ein zusätzliches Lichtfeld **9b** entsteht.

Der Hangriff 3 umfasst eine Hülse **10**, welche an der Operationsleuchte lösbar befestigt werden kann. **Fig. 4** zeigt die demontierte Hülse 10, welche über einen Flansch **11** an der Operationsleuchte angebracht werden kann. Der sterilisierbare Handgriff 3 ist zu Reinigungszwecken vom Leuchtenkörper abnehmbar. Die Arretierung und das Lösen erfolgen mithilfe eines Betätigungselements **12,** das einen Rastverschluss lösen kann. Im freien Ende der Hülse 10 sind mehrere, weißes Licht erzeugende LEDs 8 untergebracht, so dass Licht aus der Hülse 10 austreten kann. Das Licht kann dann durch eine Bohrung im Handgriff, durch ein transparentes Fenster, das ggf. die Bohrung abdeckt oder durch einen Griff aus transparentem Material austreten. Zur parallelen Strahlführung sind den LEDs 8 Linsenelemente zugeordnet. Die Zusatzbeleuchtung kann im Bedarfsfalle eingeschaltet werden.

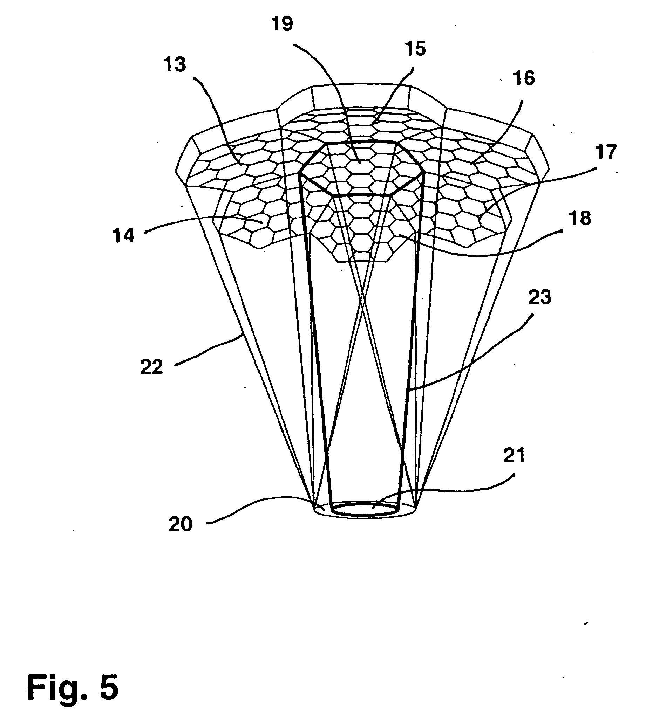

Gemäß **Fig. 5** erzeugen einzelne, beliebig kombinierbare, eine Vielzahl von LEDs umfassende Lichtmodule **13** bis **19** ein Leuchtfeld **20.** Die Lichterzeugung des zentralen Lichtmoduls 19 wird verstärkt, so dass ein zusätzliches, das Leuchtfeld 20 überlagerndes Leuchtfeld **21** entsteht, das zu Ausleuchtung tiefer Wunden verwendet wird. Mit Bezugsziffer **22** und **23** sind die Lichtstrahlen bezeichnet.

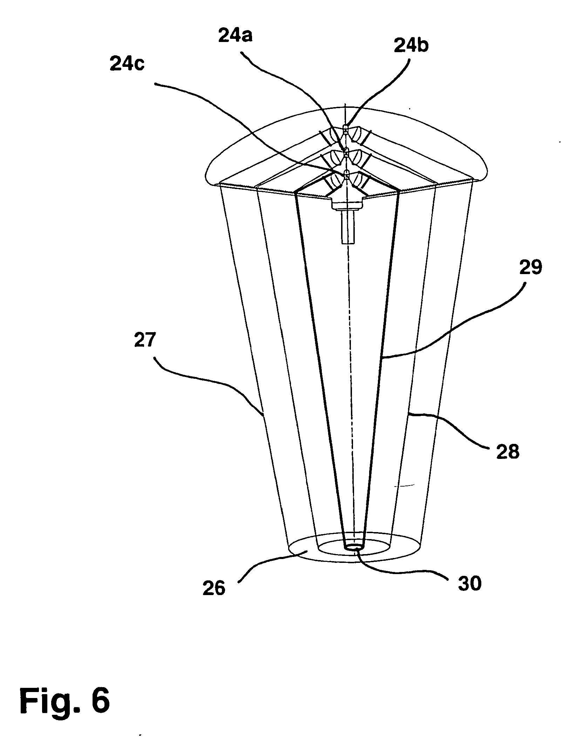

Aus der **Fig. 6** ist ersichtlich, dass eine Lichtquelle mit einzelnen von zur Aufweitung der Lichtstrahlung vorgesehenen Linsen umgebenen Leuchtmitteln **24a** bis **24c** ein Leuchtfeld **26** erzeugen. Der Strahlengang ist durch Lichtstrahlen **27** bis **29** angedeutet. Das Leuchtfeld 26 wird im Zentrum **30** verstärkt, indem die Intensität der Lichtquelle **24c** und somit der Lichtstrahlen 29 höher ist als die Strahlung im Randbereich.

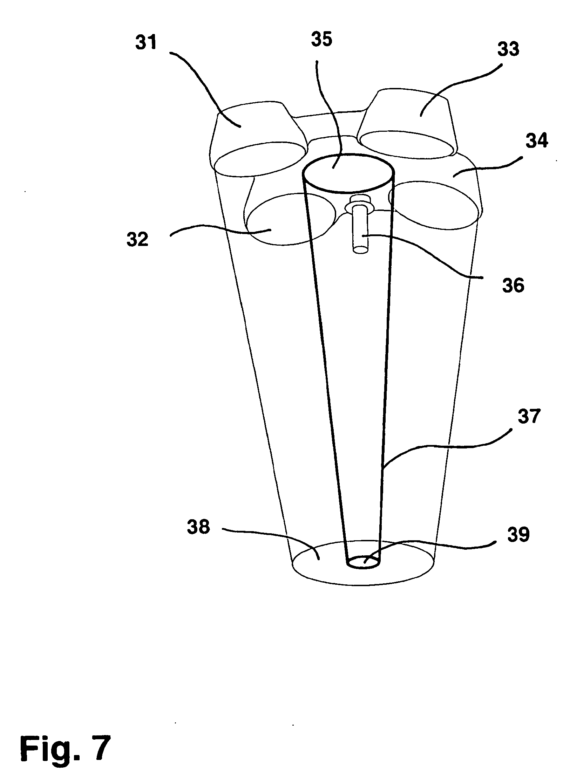

**Fig. 7** zeigt eine Operationsleuchte mit aufgelöstem, durch einzelne Leuchten **31** bis **35** ausgebildetem Lichtsystem und am Rand angebrachtem Handgriff **36.** Die zentrale Leuchte 35 erzeugt eine Lichtstrahlung **37** mit verstärkter Intensität, so dass ein Leuchtfeld **38** in seinem Zentrum **39** eine höhere Helligkeit zur Tiefenausleuchtung aufweist.

### BEZUGSZEICHENLISTE

- 1: Operationsleuchte
- 2: Leuchtenkörper
- 3: Handgriff
- 4: Element
- 5: Unterseite
- 6: Lichtquelle
- 6a: Lichtstrahl
- 6b: Lichtstrahl
- 7a: Leuchtfeld
- 7b: Leuchtfeld
- 8: Lichtquelle
- 8a: Lichttrahl
- 8b: Lichtstrahl
- 9a: Leuchtfeld
- 9b: Leuchtfeld
- 10: Hülse
- 11: Flansch
- 12: Betätigungselement
- 13: Lichtmodul
- 14: Lichtmodul
- 15: Lichtmodul
- 16: Lichtmodul
- 17: Lichtmodul
- 18: Lichtmodul
- 19: Lichtmodul
- 20: Leuchtfeld
- 21: Leuchtfeld
- 22: Lichtstrahlen
- 23: Lichtstrahlen
- 24a: Leuchtmittel
- 24b: Leuchtmittel
- 24c: Leuchtmittel
- 26: Leuchtfeld
- 27: Lichtstrahl
- 28: Lichtstrahl
- 29: Lichtstrahl
- 30: Leuchtfeld
- 31: Leuchte.
- 32: Leuchte
- 33: Leuchte
- 34: Leuchte
- 35: Leuchte
- 36: Handgriff
- 37: Lichtstrahl
- 38: Leuchtfeld
- 39: Leuchtfeld

## Patentansprüche

1. Operationsleuchte (1) mit einem Leuchtenkörper (2) zur Aufnahme von Leuchtmitteln, deren Lichtstrahlen (6a, 8a, 22, 27, 28) ein Lichtfeld (7a, 9a, 20, 26, 38) bilden, und einer Lichtquelle (6; 8; 19; 24c; 35) im Zentrum der Operationsleuchte, die unabhängig von den weiteren Leuchtmitteln ansteuerbar ist, **dadurch gekennzeichnet, dass** die aus dem Zentrum des Leuchtenkörpers (2) abgestrahlten Lichtstrahlen (6b, 8b, 23, 29, 37) ein zusätzliches Lichtfeld (7b, 9b, 21, 30, 39) im Zentrum des Lichtfelds (7a, 9a, 20, 26, 38) bilden.

2. Operationsleuchte nach Anspruch 1, **dadurch gekennzeichnet, dass** die Lichtquelle durch ein zentrales Lichtmodul (19) ausgebildet ist, welches über eine Helligkeitsregelung für eine zusätzliche Erhöhung der Lichtintensität verfügt.

3. Operationsleuchte nach Anspruch 1, **dadurch gekennzeichnet, dass** die Lichtquelle (6) durch eine ringförmig um einen Handgriff (3), der im Zentrum der Operationsleuchte angebracht ist, angeordnete zusätzliche Lichtquelle ausgebildet ist.

4. Operationsleuchte nach Anspruch 1, **dadurch gekennzeichnet, dass** die Lichtquelle (8) durch eine im Handgriff (3), der im Zentrum der Operationsleuchte angebracht ist, angeordnete zusätzliche Lichtquelle ausgebildet ist.

5. Operationsleuchte nach Anspruch 4, **dadurch gekennzeichnet, dass** die Lichtquelle in einer abnehmbaren Hülse (10) des Handgriffs (3) untergebracht ist.

6. Operationsleuchte nach einem der vorhergehenden Ansprüche, **dadurch gekennzeichnet, dass** die Lichtquelle durch LEDs ausgebildet ist.

7. Operationsleuchte nach einem der vorhergehenden Ansprüche, **dadurch gekennzeichnet, dass** Linsen zur Aufweitung des Lichtes bis zur Lichtaustrittsfläche und dann zur Lenkung von dort in das Leuchtfeld und/oder zur Vergleichmäßigung des LED-Lichts vorgesehen sind.

## Claims

1. Surgical lamp (1) with a lamp body (2) for accommodating illuminants, the light beams (6a, 8a, 22, 27, 28) of which form a light field (7a, 9a, 20, 26, 38), and a light source (6; 8; 19; 24c; 35) in the centre of the surgical lamp, the light source (6; 8; 19; 24c; 35) is controllable independently from the further illuminants, **characterized in that** the light beams (6b, 8b, 23, 29, 37) emitted out of the centre of the lamp body (2) form an additional light field (7b, 9b, 21, 30, 39) in the centre of the light field (7a, 9a, 20, 26, 38).

2. Surgical lamp according to claim 1, **characterized in that** the light source is formed by a central light module (19) having a brightness control for an additional increase of the light intensity.

3. Surgical lamp according to claim 1, **characterized in that** the light source (6) is formed by an additional light source arranged annularly about a handle (3) attached in the centre of the surgical lamp.

4. Surgical lamp according to claim 1, **characterized in that** the light source (8) is formed by an additional light source arranged in the handle (3) attached in the centre of the surgical lamp.

5. Surgical lamp according to claim 4, **characterized in that** the light source is housed in a detachable sleeve (10) of the handle (3).

6. Surgical lamp according to any of the preceding claims, **characterized in that** the light source is formed by LEDs.

7. Surgical lamp according to any of the preceding claims, **characterized in that** lenses for widening the light till the light emergence face and, then, for guiding from there into the light field and/or for homogenizing the LED-light are provided.

## Revendications

1. Scialytique (1) comprenant un corps de lampe (2) destiné au logement d'ampoules, dont les faisceaux lumineux (6a, 8a, 22, 27, 28) forment un champ lumineux (7a, 9a, 20, 26, 38) et d'une source lumineuse (6 ; 8 ; 19 ; 24c ; 35) au centre du scialytique, laquelle peut être commandée indépendamment des autres ampoules, **caractérisé en ce que** les faisceaux lumineux (6b, 8b, 23, 29, 37) diffusés depuis le centre du corps de lampe (2) forment un champ lumineux additionnel (7b, 9b, 21, 30, 39) au centre du champ lumineux (7a, 9a, 20, 26, 38).

2. Scialytique selon la revendication 1, **caractérisé en ce que** la source lumineuse est réalisée par un module lumineux central (19), lequel dispose d'un réglage de luminosité pour une élévation complémentaire de l'intensité lumineuse.

3. Scialytique selon la revendication 1, **caractérisé en ce que** la source lumineuse (6) est réalisée par une source lumineuse complémentaire, disposée en forme d'anneau autour d'une poignée (3) montée au centre du scialytique.

4. Scialytique selon la revendication 1, **caractérisé en ce que** la source lumineuse (8) est réalisée par une source lumineuse complémentaire disposée dans la poignée (3) montée au centre du scialytique.

5. Scialytique selon la revendication 4, **caractérisé en ce que** la source lumineuse est logée dans une gaine (10) amovible de la poignée (3).

6. Scialytique selon l'une des revendications précédentes, **caractérisé en ce que** la source lumineuse est réalisée par des diodes électroluminescentes.

7. Scialytique selon l'une des revendications précédentes, **caractérisé en ce que** des lentilles sont prévues pour une divergence lumineuse jusqu'à la surface de sortie de la lumière puis pour la déviation de celle-ci vers le champ lumineux, et/ou pour l'homogénéisation de la lumière des diodes électroluminescentes.
